# EUROPEAN PATENT SPECIFICATION

(11) **EP 2 309 505 B1**
(45) Date of publication and mention of the grant of the patent: **19.09.2012**
(21) Application number: 09800481.5
(22) Date of filing: 24.07.2009
(51) Int. Cl.: G11B 7/244, B41M 5/26, C07D 405/12, C07D 417/12, C09B 45/18, C09B 45/20, C09B 45/22, C09B 45/24

(54) **COLORING MATTER FOR OPTICAL INFORMATION RECORDING MEDIUM AND OPTICAL INFORMATION RECORDING MEDIUM**
FÄRBUNGSMATERIAL FÜR EIN OPTISCHES INFORMATIONSAUFZEICHNUNGSMEDIUM UND OPTISCHES INFORMATIONSAUFZEICHNUNGSMEDIUM
COLORANT POUR SUPPORT D'ENREGISTREMENT D'INFORMATIONS OPTIQUE ET SUPPORT D'ENREGISTREMENT D'INFORMATIONS OPTIQUE

(30) Priority: 25.07.2008 JP 2008192746
(43) Date of publication of application: 13.04.2011
(73) Proprietor: Taiyo Yuden Co., Ltd., Tokyo 110-0005 (JP)
(72) Inventor: KODAIRA, Takuo, Tokyo 110-0005 (JP); TSUZUKI, Takeo, Tokyo 110-0005 (JP)
(74) Representative: Müller-Boré & Partner Patentanwälte
(86) International application number: PCT/JP2009/063617
(87) International publication number: WO 2010/010970

(56) References cited:
- EP-A1- 1 903 561
- JP-A- 2007 045 147
- JP-A- 2007 196 661

## Description

### Technical Field

The present invention relates to an optical information recording medium and an organic dye used for optical information recording medium, and more specifically to a dye for optical information recording medium ideal for recordable Blu-ray Discs (BD-Rs) adopting In-Groove recording, as well as an optical information recording medium using the same.

### Prior Art

Optical discs and other optical information recording media are becoming popular as information recording media. These optical information recording media include recordable CDs (CD-Rs), each constituted by a light-transmissive resin substrate of 1.2 mm in thickness and 120 mm or 80 mm in diameter on which a reflective layer and recording layer are formed successively. In recent years, however, the market is demanding higher information recording densities. One way to respond to this demand is to use a short laser wavelength and an object lens having high numerical apertures (NAs), and this concept has led to development of new optical information recording media such as recordable DVDs (DVD±Rs). These DVD±Rs have a structure whereby two light-transmissive resin substrates of 0.6 mm in thickness are laminated together by sandwiching a reflective layer and recording layer in between, in order to increase the permissible tilt angle of the disc to accommodate a shorter wavelength and higher NAs.

In recent years, however, even higher information recording densities are required to record high-definition image data. Accordingly, optical information recording media that use laser beams of even shorter wavelengths to record and playback data are proposed. These new optical information recording media include recordable HD_DVDs (HD_DVD-Rs) having a recording capacity of 15 GB per side, as well as recordable Blu-ray Discs (BD-Rs) having a recording capacity of 25 GB per side.

A HD_DVD-R comprises a resin substrate of 0.6 mm in thickness, having a guide groove (pre-groove) provided on one side. A recording layer and reflective layer are formed successively on this substrate, and a protective layer is provided on top to protect the reflective layer. On top of this reflective layer, a dummy substrate of 0.6 mm in thickness is attached. This disc structure itself is the same as that of a DVD±R. A BD-R comprises a resin substrate of 1.1 mm in thickness, also having a guide groove provided on one side. A reflective layer and recording layer are formed successively on this substrate, and a protective layer made of light-transmissive inorganic material is provided on top to protect the recording layer. On top of this protective layer, a cover layer made of light-transmissive resin and having a thickness of 0.1 mm is provided, with the diameter and thickness of the disc adjusted to levels equivalent to those of CD-Rs and DVD±Rs. With these optical recording media, the recording layer is made of an organic substance containing azo dye, cyanine dye or other organic dye, or inorganic substance such as Si, Cu, Sb, Te or Ge, and is used to record/playback data using laser beam of 405 nm in wavelength.

If an organic dye is used in the recording layer, a dye that can provide good recording characteristics with laser beams of 405 nm in wavelength is selected. These ideal dyes for optical information recording media include those disclosed in Japanese Patent Laid-open No. 2007-45147 and Japanese Patent Laid-open No. 2007-196661.

- Patent Literature 1: Japanese Patent Laid-open No. 2007-45147
- Patent Literature 2: Japanese Patent Laid-open No. 2007-196661

### Summary of the Invention

### Problems to Be Solved by the Invention

Basically the dyes disclosed in the aforementioned known literatures can be used for both HD_DVD-Rs and BD-Rs. After earnest examinations by the inventors, however, it was revealed that optimal dye characteristics would vary depending on the recording method, or specifically between On-Groove recording and In-Groove recording. With HD_DVD-Rs, the guide groove is projecting toward the laser beam irradiation side. Accordingly, the recording method of HD_DVD-Rs is On-Groove recording. Under On-Groove recording, light concentrates more than the spot size of the laser beam. For this reason, pits formed by irradiating recording laser beams are subject to relatively less shape instability and offer relatively good recording characteristics including jitter characteristics. With BD-Rs, on the other hand, the guide groove is made concave toward the laser beam irradiation side. Accordingly, the recording method of BD-Rs is In-Groove recording. Under In-Groove recording, light does not concentrate unlike with On-Groove recording. Also with BD-Rs, the track pitch, or interval of guide grooves, is just 0.32 µm which is smaller than the laser beam wavelength of 405 nm, and consequently the spot size of the laser beam becomes greater than the guide groove. For this reason, the problem of unstable pit shapes and consequent difficulty achieving good recording characteristics has been reported.

To ensure good recording characteristics with In-Groove recording, recording must be possible at high recording sensitivity, in other words at relatively low laser output, while a dye is needed that can achieve a recording layer of relatively high modulation degree and relatively low jitter characteristics. The present invention proposes an organic dye that can achieve good recording characteristics for BD-Rs adopting In-Groove recording, and it also proposes an optical information recording medium using such dye.

### Means for Solving the Problems

The present invention proposes a dye used for optical information recording medium, which is a dye for optical information recording medium containing an organic dye expressed by the chemical formula below. 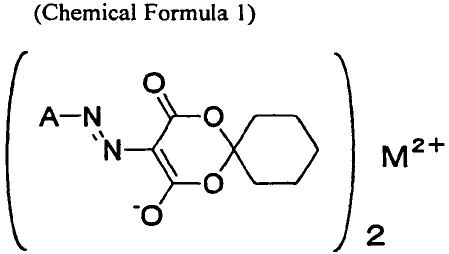 A is selected from (a1) to (a3) conforming to (Chemical Formula 2). M is selected from Ni, Co and Cu. 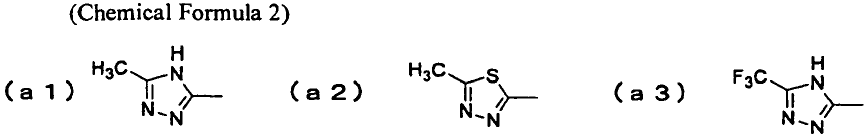 Note, however, that if (a1) is selected, Cu is excluded from the options, while if (a3) is selected, Cu and Ni are excluded from the options.

The present invention also proposes an optical information recording medium which comprises: a substrate having a helically formed guide groove on one side; a reflective layer formed on the aforementioned one side of the substrate; a recording layer formed on top of the aforementioned reflective layer; a protective layer formed on top of the aforementioned recording layer; and a light transmissive layer formed on top of the aforementioned protective layer; wherein the aforementioned recording layer contains an organic dye and an organic dye expressed by the aforementioned chemical formula is used as the organic dye.

### Effects of the Invention

According to the present invention, good recording characteristics associated with high modulation degree and low jitter characteristics can be achieved with optical information recording media whose recording method is In-Groove recording.

### Brief Description of the Drawings

Fig. 1 is a schematic diagram showing an enlarged view of the cross-section of an embodiment of an optical information recording medium.

Fig. 2 is an absorption spectrum of a dye expressed by (Chemical Formula 3).

Fig. 3 is an eye pattern of an optical information recording medium using a dye expressed by (Chemical Formula 3).

Fig. 4 is an absorption spectrum of a dye expressed by (Chemical Formula4).

Fig. 5 is an eye pattern of an optical information recording medium using a dye expressed by (Chemical Formula 4).

Fig. 6 is an absorption spectrum of a dye expressed by (Chemical Formula 5).

Fig. 7 is an eye pattern of an optical information recording medium using a dye expressed by (Chemical Formula 5).

Fig. 8 is a table summarizing the results of Examples 1 to 6 and Comparative Examples 1 to 4.

### Mode for Carrying Out the Invention

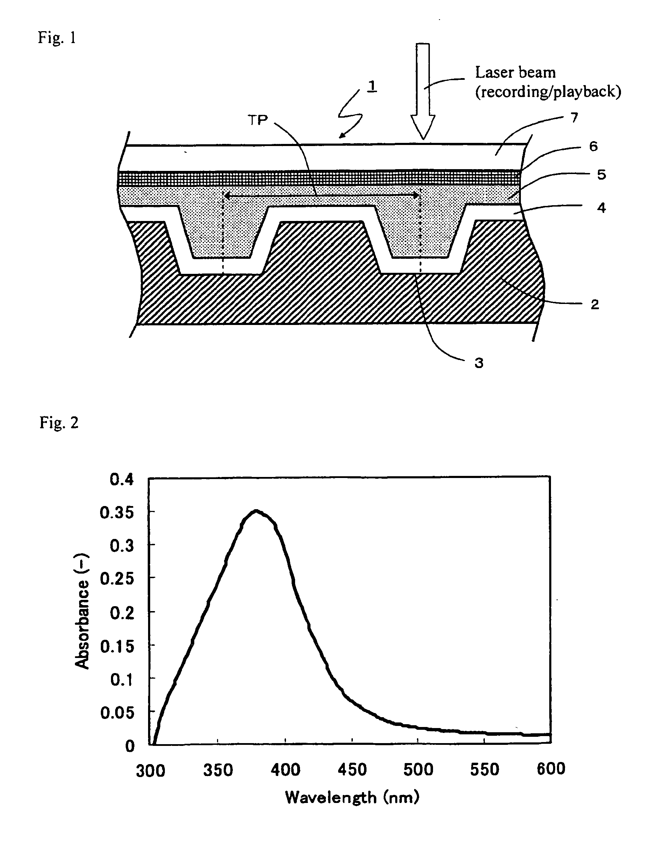

An embodiment of an optical information recording medium conforming to the present invention is explained below. The optical information recording medium 1 shown in Fig. 1 comprises: a disc-shaped substrate 2 having a through hole (not illustrated) at the center and a guide groove 3 formed helically on one side; a reflective layer 4 formed on top of the aforementioned guide groove 3 on the aforementioned substrate 2; a recording layer 5 formed on top of the aforementioned reflective layer 4 and made of an organic substance containing dye; a protective layer 6 formed on top of the aforementioned recording layer 5; and a light transmissive layer 7 formed on top of the aforementioned protective layer 6.

The substrate 2 is a resin substrate of 1.1 mm in thickness (t) and 120 mm in diameter. For this substrate 2, any of the various materials that are used as substrate materials for conventional optical information recording media can be selected and used. Specific examples include polycarbonate, polymethyl methacrylate and other acrylic resins, polyvinyl chloride, vinyl chloride copolymers and other vinyl chloride resins, epoxy resins, amorphous polyolefins, polyester resins, aluminum and other metals, and glass, among others. They can be combined or mixed or otherwise used in combination, if necessary. Among the aforementioned materials, thermoplastic resins are preferable for their formability, moisture resistance, dimensional stability, low cost, etc., and polycarbonate is especially preferable. Such substrate 2 is formed by injection molding. During the forming process, a stamper is set in the dies and this stamper forms a helical guide groove 3 on the substrate 2. This guide groove 3 is formed with a track pitch (TP) of 0.32 µm, and pits are formed inside the guide groove 3.

The reflective layer 4 is made of a thin film of a highly reflective metal such as Ag alloy or A1 alloy, and formed by sputtering, etc. A desired thickness of the reflective layer 4 is 55 nm to 65 nm.

The recording layer 5 contains the dye proposed by the present invention. The organic dye proposed by the present invention is specifically one expressed by the chemical formula shown in (Chemical Formula 3) to (Chemical Formula 8) below.

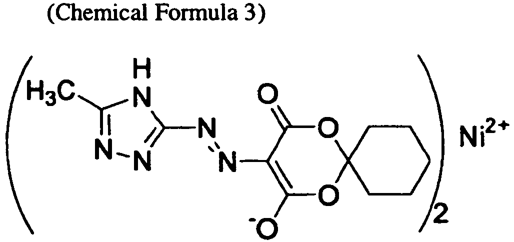

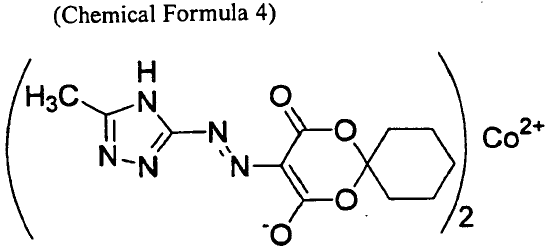

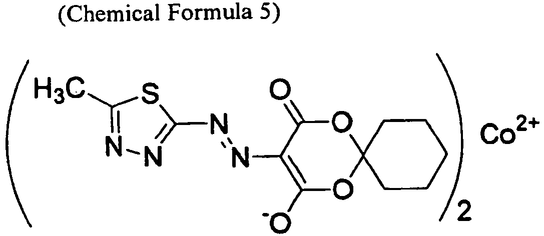

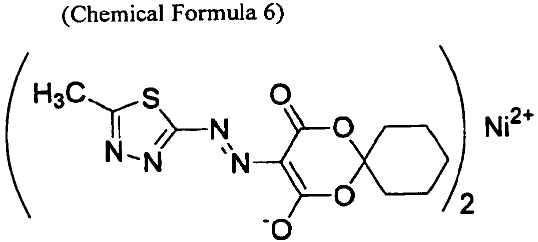

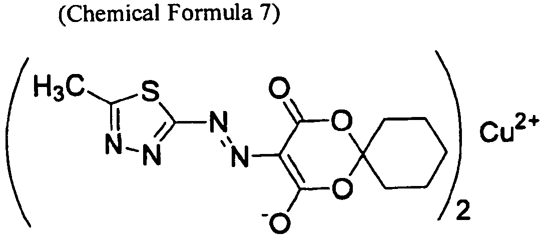

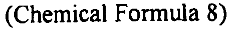

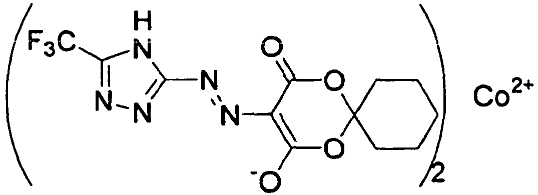

Any of these dyes can be dissolved in a TFP (tetrafluoropropanol) solution and the resulting dye solution can be applied using the spin-coat method in such a way as to achieve the optical density (hereinafter referred to as "OD") at which the DC jitter becomes the smallest, to form the recording layer. Here, the OD represents the absorbance of a dye at its maximum absorption wavelength. For the measurement method, the recording layer 5 is applied directly on the substrate 2 in Fig. 1 and light having the maximum absorption wavelength for each dye is used to measure the absorbance. For example, a dye conforming to (Chemical Formula 3) has the absorption spectrum shown in Fig. 2. Since the maximum absorption wavelength (λmax) is 379 nm, the absorbance is measured using light of 379 nm in wavelength to derive the OD. The OD is adjusted by the film forming conditions (rotational speed, time, etc.). The OD at which the DC jitter becomes the smallest is determined by preparing multiple sample discs whose recording layer 5 was made under different film forming conditions to achieve different ODs, recording data to the discs using a commercial recording/playback apparatus (such as ODU-1000 by Pulstec Industrial Co., Ltd.), and then obtaining each DC jitter and analyzing the obtained results. For example, the measured OD was 0.25 when the dye conforming to (Chemical Formula 3) was used. Spin-coating is performed under film forming conditions that give the OD determined above, to form the recording layer 5.

The protective layer 6 is provided to prevent intermixing, such as diffusion of the dye contained in the recording layer 5 to the light transmissive layer 7 when the light transmissive layer 7 is formed, or permeation into the recording layer 5 of a solvent for curable resin or other additive used in the formation of the light transmissive layer 7. The protective layer 6 may be made of, for example, silicon oxide, especially silicon dioxide, zinc oxide, cerium oxide, yttrium oxide or other oxide; zinc sulfide, yttrium sulfide or other sulfide; silicon nitride or other nitride; silicon carbide; or mixture of oxide and sulfur compound, among others. This protective layer 6 is formed by sputtering or other method. A desired thickness of the protective layer 6 is approx. 20 nm.

The light transmissive layer 7 is made of a light-transmissive resin and formed by using the spin-coat method, etc., to adjust to a thickness of 0.1 mm a resin that hardens due to UV light or radiation, or by attaching a transparent resin of 0.1 mm in thickness. The light transmittance of this light transmissive layer 6 should be 70% or more, or preferably 80% or more, when measured by a spectrophotometer based on a thickness of 0.1 mm and using light of 405 nm in wavelength. The light transmissive layer 7 is relatively soft and easy to scratch, so a hard coat layer (not illustrated) constituted by an acrylic resin, etc., may be provided on the surface where light enters.

Now, the effects of the present invention are explained using examples.

### (Example 1)

A disc-shaped polycarbonate substrate of 120 mm in outer diameter and 1.1 mm in thickness, having a through hole at the center as well as a guide groove of 0.32 µm in track pitch, 180 nm in groove width and 32 nm in groove depth, was created by injection molding. On the side of this substrate on which the guide groove was formed, Ag alloy was applied by sputtering to form a reflective layer of 60 nm in thickness. Thereafter, a dye solution prepared by dissolving in a TFP (tetrafluoropropanol) solvent an organic dye expressed by the chemical formula in (Chemical Formula 3) was applied by the spin-coat method under film forming conditions that would give an OD of 0.25, after which the substrate was dried for 30 minutes at 80°C to form a recording layer.

Next, a transparent protective layer of ZnS-SiO₂ was formed by sputtering to a thickness of 20 nm. Then, a UV-curable resin whose modulus of elasticity would become 1700 MPa at 25°C after being cured was applied by the spin-coat method, after which the resin was cured to form a light transmissive layer of 0.1 mm in thickness.

On the optical information recording medium thus obtained, pits were recorded in the guide groove using a recording/playback apparatus (ODU-1000 by Pulstec Industrial Co., Ltd.) at a wavelength of 405 nm, NA of 0.85 and recording speed of 9.84 m/s, to evaluate the playback characteristics. The evaluation items included the power of the recording laser beam (recording power), modulation degree, and DC jitter. For the recording power, the power at which the DC jitter became the smallest was measured, and any value equal to or below 6.0 mW was determined as an indication of good recording sensitivity. For the modulation degree, 40% or more was deemed acceptable. For the DC jitter, the minimum value of 10% or less was deemed acceptable.

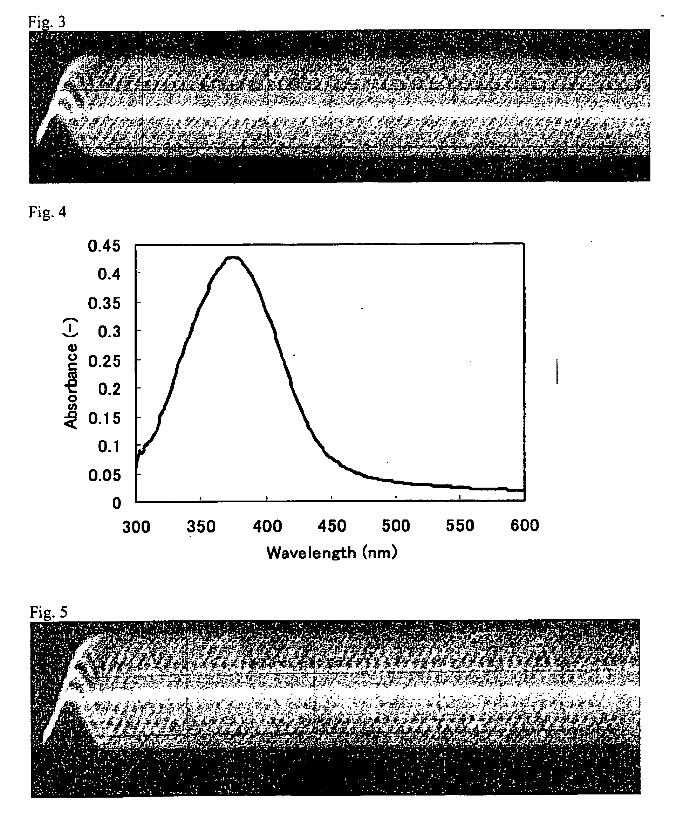

The optical information recording medium obtained in Example 1 had a recording power of 4.7 mW, modulation degree of 45% and DC jitter of 7.8%. When the laser power was adjusted to 0.35 mW and data was played back, the clear eye pattern shown in Fig. 3 was observed.

### (Example 2)

An optical information recording medium was obtained in the same manner as in Example 1, except that an organic dye expressed by the chemical formula in (Chemical Formula 4) was used for the recording layer, instead of the organic dye in Example 1. This dye conforming to (Chemical Formula 4) had the absorption spectrum shown in Fig. 4. Since the maximum absorption wavelength (λmax) of this dye was 375 nm, the absorbance was measured with light of 375 nm in wavelength to derive the OD. Because the OD at which the DC jitter became the smallest was 0.32, the recording layer was formed under film forming conditions that would give an OD of 0.32.

The sample optical information recording medium thus obtained was evaluated for its playback characteristics by recording data using a recording/playback apparatus (ODU-1000 by Pulstec Industrial Co., Ltd.) in the same manner as in Example 1. The eye pattern was also observed at a laser power of 0.35 mW. The optical information recording medium obtained in Example 2 had a recording power of 5.0 mW, modulation degree of 48% and DC jitter of 7.4%. Also, the clear eye pattern shown in Fig. 5 was observed.

### (Example 3)

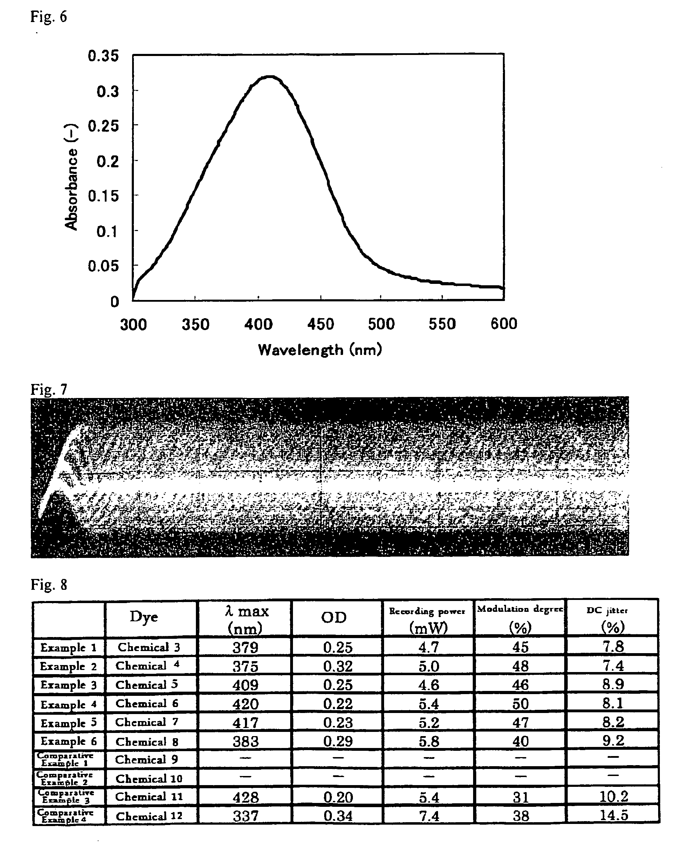

An optical information recording medium was obtained in the same manner as in Example 1, except that an organic dye expressed by the chemical formula in (Chemical Formula 5) was used for the recording layer, instead of the organic dye in Example 1. This dye conforming to (Chemical Formula 5) had the absorption spectrum shown in Fig. 6. Since the maximum absorption wavelength (λmax) of this dye was 409 nm, the absorbance was measured with light of 409 nm in wavelength to derive the OD. Because the OD at which the DC jitter became the smallest was 0.25, the recording layer was formed under film forming conditions that would give an OD of 0.25.

The sample optical information recording medium thus obtained was evaluated for its playback characteristics by recording data using a recording/playback apparatus (ODU-1000 by Pulstec Industrial Co., Ltd.) in the same manner as in Example 1. The eye pattern was also observed at a laser power of 0.35 mW. The optical information recording medium obtained in Example 3 had a recording power of 4.6 mW, modulation degree of 46% and DC jitter of 8.9%. Also, the clear eye pattern shown in Fig. 7 was observed.

### (Example 4)

An optical information recording medium was obtained in the same manner as in Example 1, except that an organic dye expressed by the chemical formula in (Chemical Formula 6) was used for the recording layer, instead of the organic dye in Example 1. Since the maximum absorption wavelength (λmax) of this dye conforming to (Chemical Formula 6) was 420 nm, the absorbance was measured with light of 420 nm in wavelength to derive the OD. Because the OD at which the DC jitter became the smallest was 0.22, the recording layer was formed under film forming conditions that would give an OD of 0.22.

The sample optical information recording medium thus obtained was evaluated for its playback characteristics by recording data using a recording/playback apparatus (ODU-1000 by Pulstec Industrial Co., Ltd.) in the same manner as in Example 1. The optical information recording medium obtained in Example 4 had a recording power of 5.4 mW, modulation degree of 50% and DC jitter of 8.1%.

### (Example 5)

An optical information recording medium was obtained in the same manner as in Example 1, except that an organic dye expressed by the chemical formula in (Chemical Formula 7) was used for the recording layer, instead of the organic dye in Example 1. Since the maximum absorption wavelength (λmax) of this dye conforming to (Chemical Formula 7) was 417 nm, the absorbance was measured with light of 417 nm in wavelength to derive the OD. Because the OD at which the DC jitter became the smallest was 0.23, the recording layer was formed under film forming conditions that would give an OD of 0.23.

The sample optical information recording medium thus obtained was evaluated for its playback characteristics by recording data using a recording/playback apparatus (ODU-1000 by Pulstec Industrial Co., Ltd.) in the same manner as in Example 1. The optical information recording medium obtained in Example 5 had a recording power of 5.2 mW, modulation degree of 47% and DC jitter of 8.2%.

### (Example 6)

An optical information recording medium was obtained in the same manner as in Example 1, except that an organic dye expressed by the chemical formula in (Chemical Formula 8) was used for the recording layer, instead of the organic dye in Example 1. Since the maximum absorption wavelength (λmax) of this dye conforming to (Chemical Formula 8) was 383 nm, the absorbance was measured with light of 383 nm in wavelength to derive the OD. Because the OD at which the DC jitter became the smallest was 0.29, the recording layer was formed under film forming conditions that would give an OD of 0.29.

The sample optical information recording medium thus obtained was evaluated for its playback characteristics by recording data using a recording/playback apparatus (ODU-1000 by Pulstec Industrial Co., Ltd.) in the same manner as in Example 1. The optical information recording medium obtained in Example 6 had a recording power of 5.8 mW, modulation degree of 40% and DC jitter of 9.2%.

### (Comparative Example 1)

An attempt was made to use, in Example 1, an organic dye expressed by the chemical formula in (Chemical Formula 9) below to form the recording layer. However, this dye did not dissolve in TFP, ethanol or 2-methoxy ethanol, etc., and film could not be formed, and therefore a sample disc could not be created or evaluated.

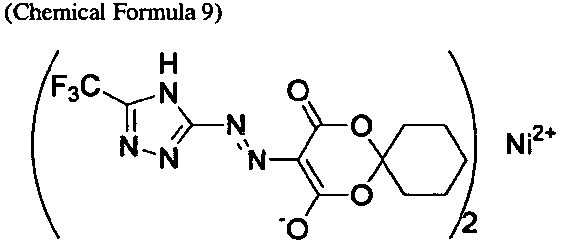

### (Comparative Example 2)

An attempt was made to use, in Example 1, an organic dye expressed by the chemical formula in (Chemical Formula 10) below to form the recording layer. However, this dye did not dissolve in TFP, ethanol or 2-methoxy ethanol, etc., and film could not be formed, and therefore a sample disc could not be created or evaluated.

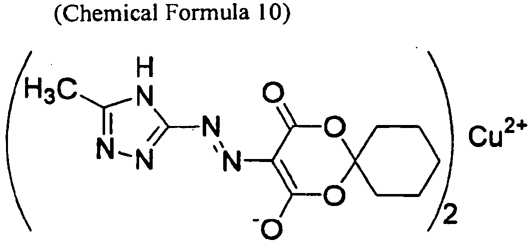

### (Comparative Example 3)

An optical information recording medium was obtained in the same manner as in Example 1, except that an organic dye expressed by the chemical formula in (Chemical Formula 11) below was used for the recording layer, instead of the organic dye in Example 1. Since the maximum absorption wavelength (λmax) of this dye conforming to (Chemical Formula 11) was 428 nm, the absorbance was measured with light of 428 nm in wavelength to derive the OD. Because the OD at which the DC jitter became the smallest was 0.20, the recording layer was formed under film forming conditions that would give an OD of 0.20.

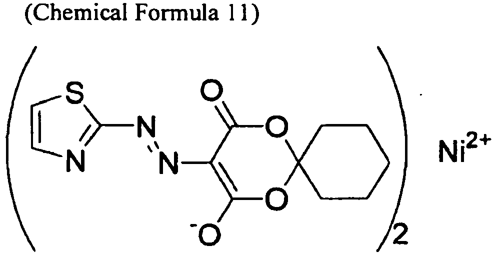

The sample optical information recording medium thus obtained was evaluated for its playback characteristics by recording data using a recording/playback apparatus (ODU-1000 by Pulstec Industrial Co., Ltd.) in the same manner as in Example 1. The optical information recording medium obtained in Example 4 had a recording power of 5.4 mW, modulation degree of 31% and DC jitter of 10.2%.

### (Comparative Example 4)

An optical information recording medium was obtained in the same manner as in Example 1, except that an organic dye expressed by the chemical formula in (Chemical Formula 12) below was used for the recording layer, instead of the organic dye in Example 1. Since the maximum absorption wavelength (λmax) of this dye conforming to (Chemical Formula 12) was 337 nm, the absorbance was measured with light of 337 nm in wavelength to derive the OD. Because the OD at which the DC jitter became the smallest was 0.34, the recording layer was formed under film forming conditions that would give an OD of 0.34.

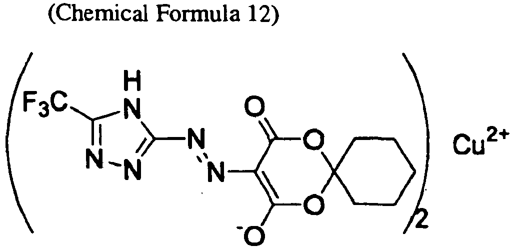

The sample optical information recording medium thus obtained was evaluated for its playback characteristics by recording data using a recording/playback apparatus (ODU-1000 by Pulstec Industrial Co., Ltd.) in the same manner as in Example 1. The optical information recording medium obtained in Example 4 had a recording power of 7.4 mW, modulation degree of 38% and DC jitter of 14.5%.

Fig. 8 is a table summarizing the aforementioned results. These results show that by using a dye conforming to the present invention, an optical information recording medium offering good recording characteristics can be obtained. In addition, since the sample in each of the aforementioned examples has the same structure as that of BD-Rs, a dye conforming to the present invention is suitable for optical information recording media whose recording method is In-Groove recording.

### Description of the Symbols

- 1: Optical information recording medium
- 2: Substrate
- 3: Guide groove
- 4: Reflective layer
- 5: Recording layer
- 6: Protective layer
- 7: Light transmissive layer
- TP: Track pitch

## Claims

1. A dye for optical information recording medium, **characterized in that** it is used for optical information recording medium and contains an organic dye expressed by the chemical formula below: 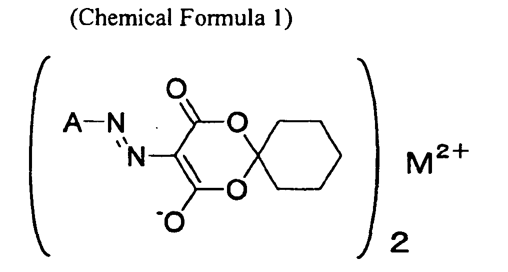 wherein A is selected from (a1) to (a3) conforming to (Chemical Formula 2), while M is selected from Ni, Co and Cu: 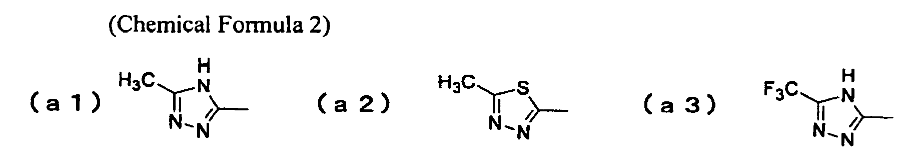 where if (a1) is selected, Cu is excluded from the options, while if (a3) is selected, Cu and Ni are excluded from the options.

2. An optical information recording medium comprising: a substrate having a helically formed guide groove on one side; a reflective layer formed on the one side of the substrate; a recording layer formed on top of the reflective layer; a protective layer formed on top of the recording layer; and a light transmissive layer formed on top of the protective layer; said optical information recording medium **characterized in that** the recording layer contains an organic dye and an organic dye expressed by the chemical formula below is used for the organic dye: 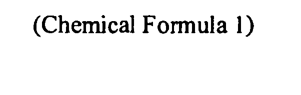 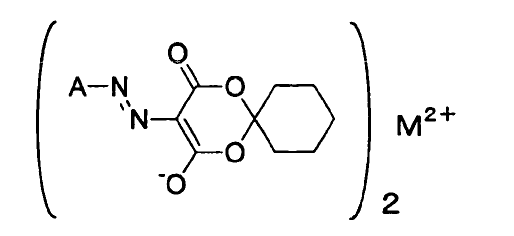 wherein A is selected from (a1) to (a3) conforming to (Chemical Formula 2), while M is selected from Ni, Co and Cu: 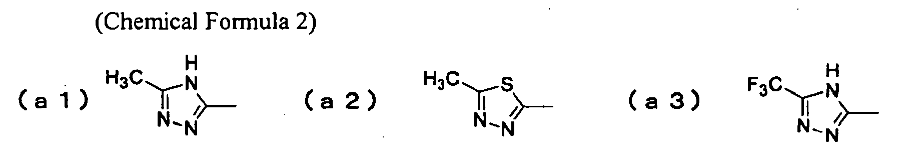 where if (a1) is selected, Cu is excluded from the options, while if (a3) is selected, Cu and Ni are excluded from the options.

## Patentansprüche

1. Farbstoff für ein optisches Informationsaufzeichnungsmedium, **dadurch gekennzeichnet, dass** dieser für ein optisches Informationsaufzeichnungsmedium verwendet wird und einen organischen Farbstoff, dargestellt durch die untenstehende chemische Formel: 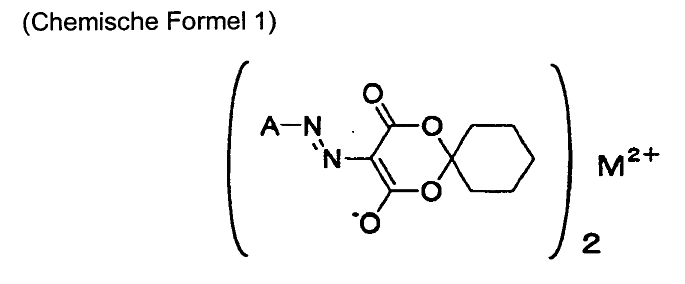 enthält,
wobei A aus (a1) bis (a3), übereinstimmend mit (Chemische Formel 2), ausgewählt ist, wobei M aus Ni, Co und Cu ausgewählt ist: 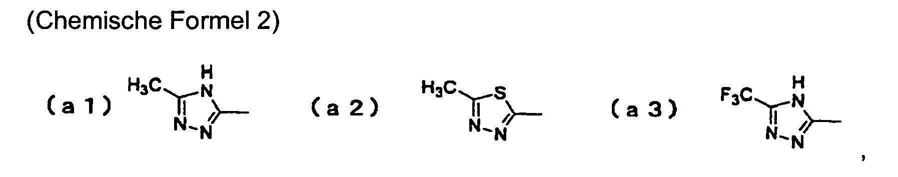 wobei, wenn (a1) ausgewählt ist, Cu von den Optionen ausgeschlossen ist, wobei, wenn (a3) ausgewählt ist, Cu und Ni von den Optionen ausgeschlossen sind.

2. Optisches Informationsaufzeichnungsmedium, umfassend:
ein Substrat mit spiralförmiger Führungsrille auf einer Seite,
eine Reflexionsschicht, gebildet auf der einen Seite des Substrats,
eine Aufzeichnungsschicht, gebildet oberseitig der Reflexionsschicht,
eine Schutzschicht, gebildet oberseitig der Aufzeichnungsschicht und eine lichtdurchlässige Schicht, gebildet oberseitig der Schutzschicht,
wobei das optische Informationsaufzeichnungsmedium **dadurch gekennzeichnet ist, dass** die Aufzeichnungsschicht einen organischen Farbstoff enthält und ein organischer Farbstoff, dargestellt durch die untenstehende chemische Formel für den organischen Farbstoff verwendet wird: 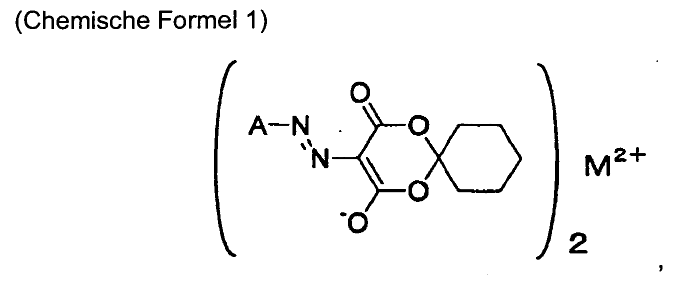
wobei A aus (a1) bis (a3), übereinstimmend mit (Chemische Formel 2), ausgewählt ist, wobei M aus Ni, Co und Cu ausgewählt ist: 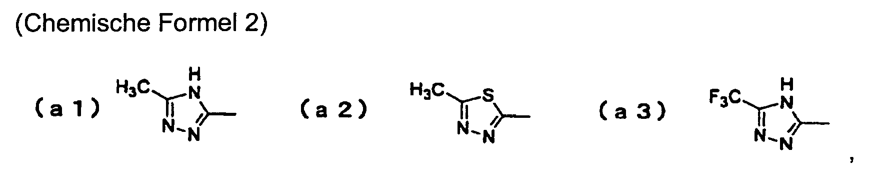
wobei, wenn (a1) ausgewählt ist, Cu von den Optionen ausgeschlossen ist, wobei, wenn (a3) ausgewählt ist, Cu und Ni von den Optionen ausgeschlossen sind.

## Revendications

1. Colorant pour support d'enregistrement d'informations optique, **caractérisé en ce qu'**il est utilisé pour un support d'enregistrement d'informations optique et **en ce qu'**il contient un colorant organique exprimé par la formule chimique ci-dessous : 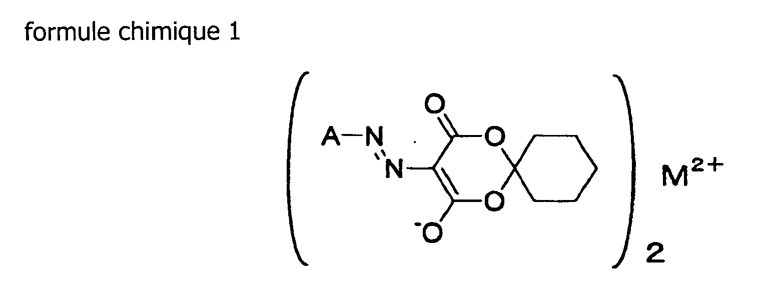 où A est choisi parmi (a1) à (a3) selon la formule chimique 2, alors que M est choisi parmi Ni, Co et Cu : 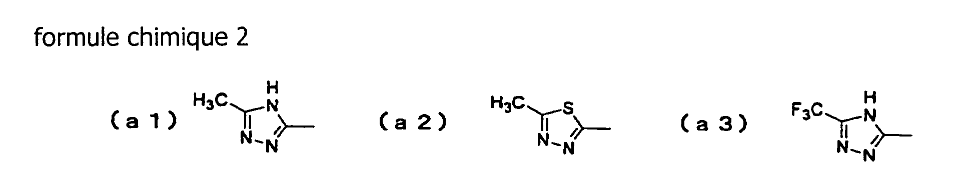 où si (a1) est choisi, Cu est exclu des options, alors que si (a3) est choisi, Cu et Ni sont exclus des options.

2. Support d'enregistrement d'informations optique comprenant un substrat ayant une rainure de guidage formée de manière hélicoïdale sur une face ; une couche de réflexion formée sur une face du substrat ; une couche d'enregistrement formée sur le haut de la couche de réflexion ; une couche protectrice formée sur le haut de la couche d'enregistrement, et une couche transmettant la lumière formée sur le haut de la couche protectrice, ledit support d'enregistrement d'informations optique étant **caractérisé en ce que** la couche d'enregistrement contient un colorant organique, et un colorant organique exprimé par la formule chimique ci-dessous est utilisé comme colorant organique : 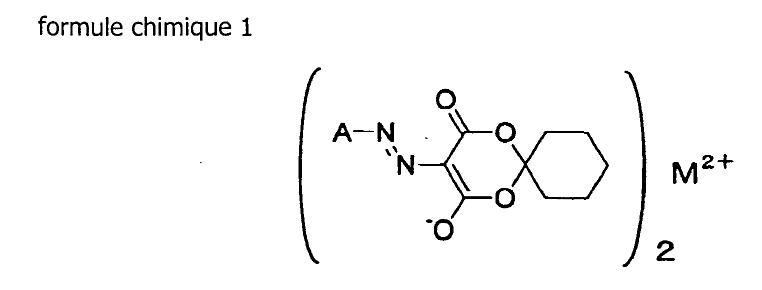 où A est choisi parmi (a1) à (a3) selon la formule chimique 2, alors que M est choisi parmi Ni, Co et Cu : 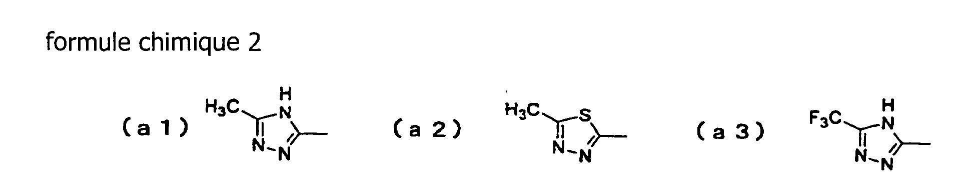 où si (a1) est choisi, Cu est exclu des options, alors que si (a3) est choisi, Cu et Ni sont exclus des options.
